# EUROPEAN PATENT SPECIFICATION

(11) **EP 1 417 001 B1**
(45) Date of publication and mention of the grant of the patent: **16.01.2008**
(21) Application number: 02757116.5
(22) Date of filing: 13.08.2002
(51) Int. Cl.: A61N 1/32, A61N 1/36

(54) **GRADUAL RECRUITMENT OF MUSCLE/NEURAL EXCITABLE TISSUE USING HIGH-RATE ELECTRICAL STIMULATION PARAMETERS**
ALLMÄHLICHE REKRUTIERUNG VON ERREGBAREM MUSKEL/NERVEN-GEWEBE UNTER VERWENDUNG ELEKTRISCHER STIMULATIONSPARAMETER MIT HOHER RATE
RECRUTEMENT PROGRESSIF DE TISSU MUSCULAIRE/NERVEUX EXCITABLE PAR UTILISATION DE PARAMETRES DE STIMULATION ELECTRIQUE HAUT DEBIT

(30) Priority: 17.08.2001 US 313223 P
(43) Date of publication of application: 12.05.2004
(73) Proprietor: ADVANCED BIONICS CORPORATION, Sylmar, CA 91342 (US)
(72) Inventor: OVERSTREET, Edward, H., Castaic, CA 91384 (US)
(74) Representative: Howe, Steven
(86) International application number: PCT/US2002/025861
(87) International publication number: WO 2003/015863

(56) References cited:
- WO-A-96/12383
- US-A- 3 562 408
- US-A- 4 495 384
- US-A- 5 016 647
- US-A- 5 758 651
- US-A- 6 078 838
- US-B1- 6 249 704

## Description

### Background of the Invention

The present invention relates to implantable neural stimulators, and more particularly to an implantable neural stimulator and a method of using such implantable neural stimulator so as to gradually recruit muscle or neural excitable tissue in a more natural and efficacious manner.

Stimulation of excitable tissues, i.e., neural or muscular, utilizing wide pulse widths, and low rates, as are commonly used in the prior art, tends to force populations of fibers within the proximity of the electrode to exhibit synchronized firing. Indeed, synchronized firing has been the goal of many of these devices because historically it was thought that excitable tissue, if it is to be stimulated, should be stimulated so as to fire synchronously. Such synchronized firing causes the excitable tissue to exhibit nearly uniform input/output firing rate functions, thereby exhibiting minimal statistical variability. Disadvantageously, however, minimal statistical variability induces unnatural firing properties. Such unnatural firing properties are unable to generate a sufficient integrated electrical dynamic range within an excitable tissue to mimic biological recruitment characteristics. It is thus seen that there is a need for a neural stimulation system and method that overcomes the limitations associated with synchronized firing and that mimics biological recruitment characteristics.

United States Patent No. 6,078,838, issued to Jay Rubinstein, teaches a particular type of pseudo-spontaneous neural stimulation system and method. The neural stimulation method taught by Rubinstein in the '838 patent generates stochastic independent activity across an excited nerve or neural population in order to produce what is referred to as "pseudo spontaneous activity". Varying rates of pseudo spontaneous activity are created by varying the intensity of a fixed amplitude, high rate pulse train stimulus, e.g., of 5000 pulses per second (pps). The pseudo spontaneous activity is said to desynchronize the nerve fiber population as a treatment for tinnitus.

United States Patent No. 6,249,704, issued to Albert Maltan et al., applies non-auditory-informative stimuli as well as auditory-informative stimuli to the same or neighboring sets of electrodes within the cochlea of a patient. The non-auditory-informative stimuli influence the properties and response characteristics of the auditory system so that when the auditory-informative stimuli are applied, such stimuli are more effective at evoking a desired auditory response, i.e., are more effective at allowing the patient to perceive sound.

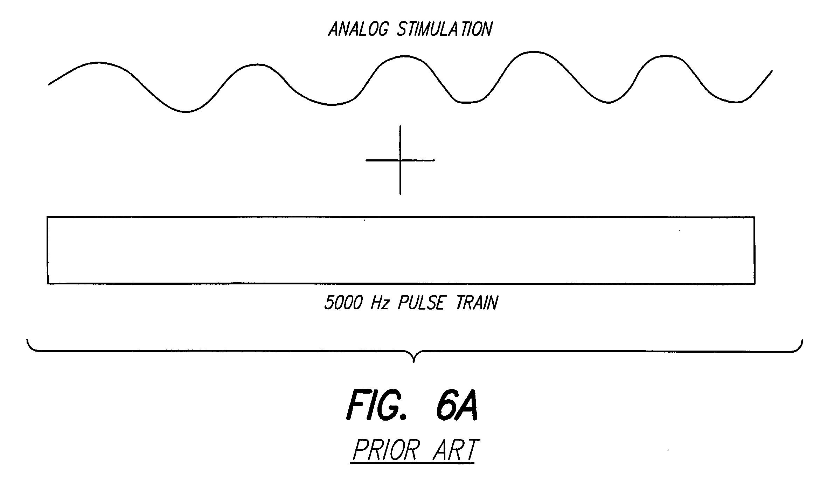

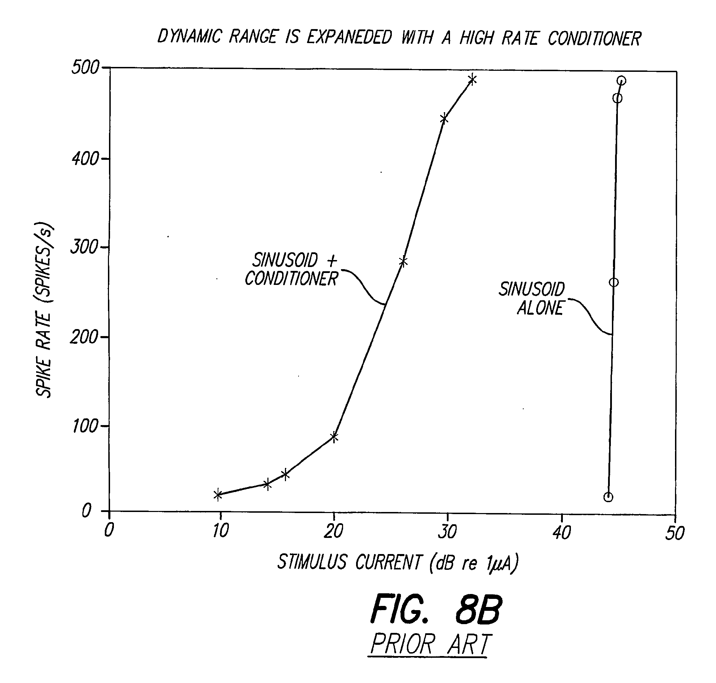

One approach known in the art for expanding the dynamic range achieved with, for example, a cochlear implant is to apply a high rate conditioning signal, e.g., a 5000 Hz pulse train, in combination with analog stimulation to the electrode contacts in contact with the inner ear tissue to be stimulated. The 5000 Hz pulse train functions as a conditioner. See, Rubinstein et al., Second Quarterly Progress Report N01-DC-6-2111 and U.S. Patent 6,078,838. This approach, and the results achieved thereby, are illustrated in FIGS. 6A and 6B. Disadvantageously, the approach proposed by Rubinstein et al. requires a painstaking process to determine the level of the non-information conditioner pulse train. Moreover, because it is combined with analog stimulation, the power consumption is exorbitantly high.

### Summary of the Invention

The present invention addresses the above and other needs by utilizing high rate (e.g., greater than 2000 Hz) pulsitile stimulation to stimulate excitable tissue. Such high rate pulsitile stimulation exploits the subtle electro physiological differences between excitable tissue cells in order to desynchronize action potentials within the population as well as to induce a wider distribution of population thresholds and electrical dynamic ranges.

The present invention overcomes the limitations brought about by synchronized, unnatural firing. The stimulation provided by the invention is configured to elicit graded muscle contractions as well as wide dynamic ranges. Such beneficial results are accomplished by utilizing electrical stimulation parameters that provide an Inefficiency of fiber recruitment similar to that seen for synaptic release of vesicular contained neurotransmitters.

The neurostimulation provided by the invention produces a wide variety of beneficial results, Including functional limb movement, wide electrical dynamic ranges for spiral ganglion cell neurons in cochlear implants, retinal ganglion cell firing patterns in visual prosthetics, as well as functional recruitment for any excitable tissue. Additional beneficial purposes made possible by the invention comprise: generating graded muscular movements, targeting class C sensory fibers for the purpose of pain relief, triggering auditory nerve fibers to provide the sensation of hearing, and/or encoding sensory information, to name but a few.

In accordance one aspect of the invention stochastic firing is restored to the excitable tissue cells, thereby enhancing thresholds, dynamic range and psycho physical performance.

In accordance with yet another aspect of the invention Individual neurons are stimulated by a neurostimulator implant at a rate faster than the individual cells are able to follow, thereby resulting in a randomization of interspike (firing) intervals. Advantageously, when the neuron is no longer phase-locked to a carrier pulse, the firing probability becomes a function of stimulus energy, and becomes much more like a "natural" biological function.

### Brief Description of the Drawings

The above and other aspects, features and advantages of the present invention will be more apparent from the following more particular description thereof, presented in conjunction with the following drawings wherein:

FIG. 1 is a current stimulation waveform that defines the stimulation rate (1/T) and biphasic pulse width (PW) associated with electrical stimuli, as those terms are used in the present application;

FIG. 2A and 2B schematically illustrate, by way of example, the hair cells in the cochlea and the nerve fiber synapse which is the origin of stochastic spontaneous firing within the cochlea;

FIG. 3 shows the average firing rate of an auditory nerve fiber as a function of IHC voltage;

FIG. 4 illustrates how dynamic range is affected by the magnitude of a modulating signal;

FIG. 5 shows how dynamic range is significantly narrowed when traditional electrical stimulation is employed;

FIG. 6A illustrates one method known in the art for inducing stochastic neural firing using a cochlear implant;

FIG. 6B shows how the method of FIG. 6A expands dynamic range;

FIG. 7A depicts an auto-conditioning with high resolution (ACHR) pulse train of the type utilized by the present invention;

FIG. 7B shows a functional block diagram of a neurostimulator configured to generate an ACHR neurostimulation signal;

FIG. 8 conceptually illustrates how auto-conditioning with high resolution neurostimulation induces stochastic neural firing of all adjacent neurons; and

FIG. 9 illustrates a spike count histogram for the ACHR neurostimulation provided by the invention.

Corresponding reference characters indicate corresponding components throughout the several views of the drawings.

### Detailed Description of the Invention

The following description is of the best mode presently contemplated for carrying out the invention. This description is not to be taken in a limiting sense, but is made merely for the purpose of describing the general principles of the invention. The scope of the invention should be determined with reference to the claims.

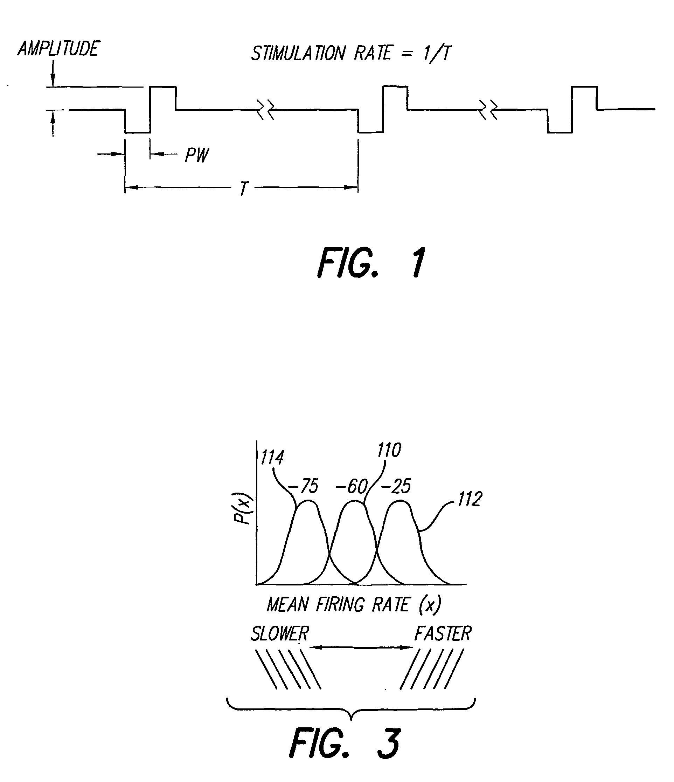

FIG. 1 shows a waveform diagram of a biphasic pulse train, and defines the stimulation period (T), stimulation rate (1/T), amplitude, and pulse width (PW) as those terms are used in the present application.

The present invention is aimed at providing gradual recruitment of muscle/neural excitable tissue through the application of a high rate electrical stimulation signal that is amplitude modulated with the desired control information. The beneficial results achieved through such stimulation occur because the stimulus pattern induces stochastic, i.e. random, neural firing, which stochastic neural firing acts to restore "spontaneous" neural activity. In fact, the high rate stimulus pattern provided by the invention stimulates individual neurons at a rate faster than the individual neuron can follow. This results in a randomization of inter-spike intervals, where the inter-spike interval is the time between successive neural firings for a given neuron; or stated differently, inter-spike intervals represent the "firing patterns" of individual nerve fibres. The inter-spike intervals, or firing patterns, of all nerve fibres within a selected group of excitable tissue, tend to be stochastic (random). Furthermore, these firing patterns are stochastic across the neural population. Advantageously, when the neuron is no longer phase-locked to a carrier pulse, as is usually the case when prior art neural stimulators are used, its firing probability becomes a function of stimulus energy, and thus becomes more like a "natural" biological function. Such randomization in a neural population better enables the population of neuron fibres to encode the fine details associated with the biological function performed by such population. That is, the population of neuron fibres is able to encode what a single neuron fibre is not able to encode.

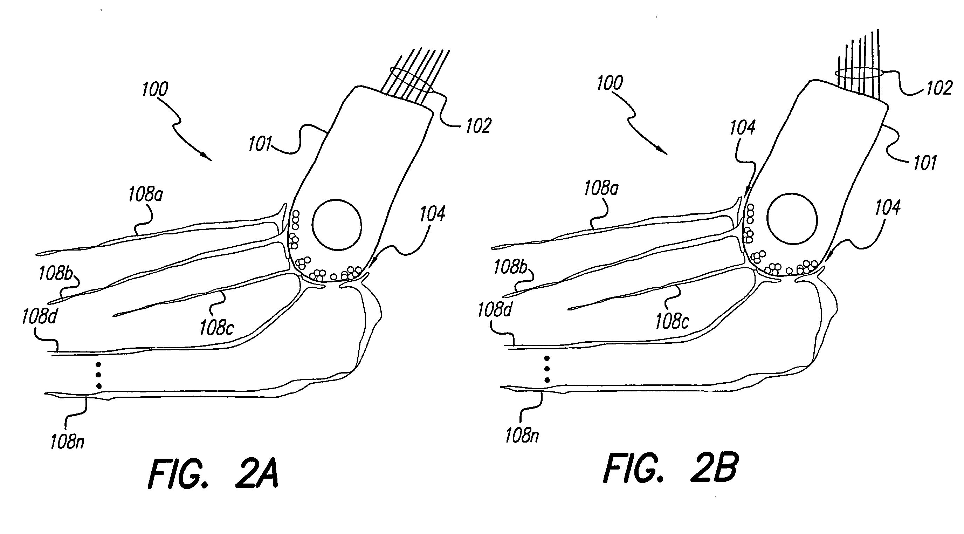

By way of illustration, the improvements obtained through randomization of the neural population will next be explained relative to transduction and neural coding within the cochlea. It is the voltage fluctuations within an inner hair cell (IHC) 101 (see FIG. 2A) that initiate the neural impulses sent to the brain through the auditory nerve that allow a person to perceive sound. The biological function performed by the IHC 101, or stated more correctly, by the population of IHC's 101 found in both left and right cochlea of a patient, is the transduction of mechanical vibration into a neural code which is interpreted by the brain as hearing. It is to be emphasised that the present invention is not related to use with an IHC 101, or population of IHCs, of the cochlea. Rather, the target to be stimulated according to the present invention is muscular and/or neural excitable tissue(s) of a limb. In particular, it is noted that where there is a bundle of muscle fibres or muscle tissue to be electrically stimulated, there exists a very narrow window from going to no response to a full recruitment of fibres. What is needed relative to muscle stimulation is a gradual response. The present invention advantageously provides for such a gradual response, either through direct electrical stimulation of the muscle fibres or tissue, or through electrical stimulation of the nerves that innervate the muscle fibres or tissue.

FIG. 2A schematically depicts an inner hair cell (IHC) nerve fibre complex 100. The IHC 101 is the transduction cell, or sensory receptor, of the cochlea. At one end of the hair cell 101 are tiny hairs 102, known as stereocilia, that are exposed on the inner surface of the cochlea. These hairs 102 move back and forth as the fluid in the cochlea moves back and forth, which movement causes a voltage to appear across the IHC membrane. (The fluid in the cochlea moves back and forth as a function of pressure waves, i.e. sound waves, sensed through the outer and middle ear or, in some instances, sensed through bone conduction.) Other types of cells or nerve fibres through the body have analogous means for sensing a particular event or condition. At the other end of the nerve fibre complex 100 are nerve fibre synapses 104. A synapse is a minute gap across which nerve impulses pass from one neuron to the next, at the end of a nerve fibre. Reaching a synapse, an impulse causes the release of a neurotransmitter, which diffuses across the gap and triggers an electrical impulse in the next neuron. In a healthy ear, the movement of the hairs or stereocilia 102 causes a nerve impulse to pass through the fibre complex 100 to the synapse 104. The nerve fibre synapse 104 is the origin of stochastic spontaneous firing. The nerve fiber synapses 104 are coupled to individual auditory nerve fibers 108a, 108b, 108c, 108d, ... 108n, which nerve fibers, in turn, are coupled through ganglion cell bodies to the cochlear nerve, which forms part of the vestibulocochlear nerve (cranial nerve VIII) connecting with the brain. It is technically not correct to refer to the IHC 101 as being "fired" because, as indicated above, "firing" occurs at the nerve fiber synapse 104, not in the IHC 101. The auditory nerve fiber 108 is thus the "spiking cell", where the effect of a nerve impulse passing through a synapse 104 is manifest. It is not uncommon, as sort of a short-hand way of referencing the chain of events that causes an auditory or other nerve fiber to become spiked as the "firing" of a nerve cell, e.g., the firing of a ganglion cell, or simply as "neural firing".

FIG. 2B shows that as the IHC membrane voltage changes, i.e., as the stereocilia 102 are displaced, the probability of transmitter release also changes (but the release is still random) as a function of stimulus energy. At any instant of time, in response to sensed sound that causes the stereocilia 102 to be displaced, or in response to silence, where the stereocilia 102 remain substantially at rest, the nerve fiber synapses 104 fire in a stochastic (random) manner, causing nerve impulses to be sent along the respective auditory nerve fibers. As the stimulus energy increases, the probability that more nerve fibers will fire increases, but the firing remains stochastic, or random.

Therefore, when electrical stimulation is provided through the use of a cochlear implant device --and it is to be noted that in most instances where a cochlear implant device is used, it is because the IHC has been lost - such implant device, in order to better represent a "natural" biological function, should induce a stimulus randomness like that of the healthy IHC. The present invention advantageously focuses on achieving such stimulation randomness.

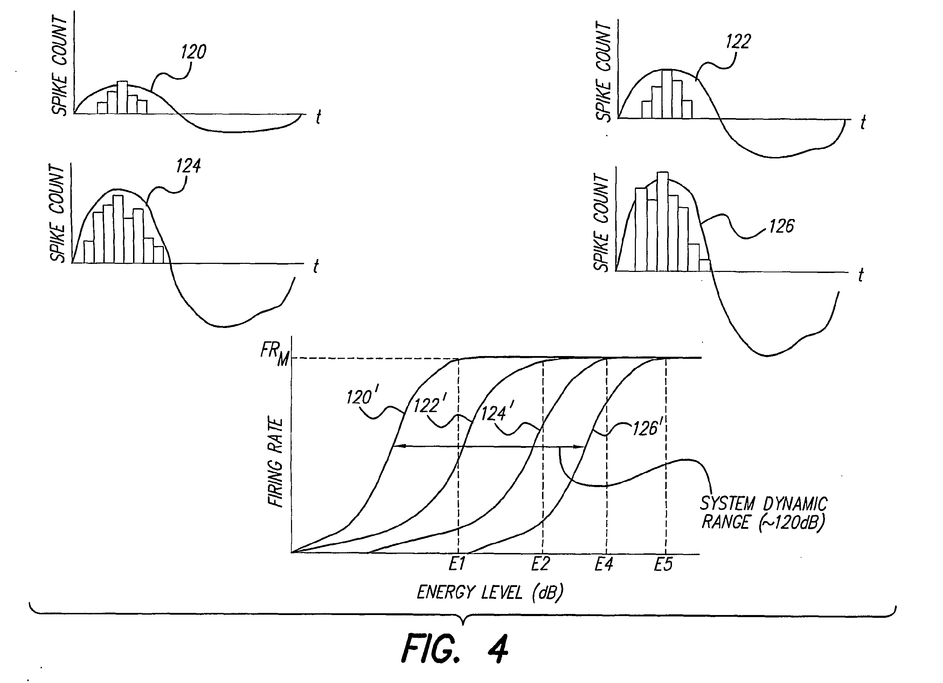

The curve 110 in FIG. 3 shows the average firing rate of an auditory nerve fiber as a function of the IHC voltage when the IHC is at rest (and the IHC voltage is about -60 mV). As seen in FIG. 3, such average firing rate has a probability distribution P(X) about a mean firing rate (X).

FIG. 3 also shows, as curve 112, the average firing rate of an auditory nerve fiber as a function of the IHC voltage when the IHC is depolarized, i.e., when the stereocilia 102 have been displaced in one direction, and the IHC voltage is about -25 mV. As seen in FIG. 3, in such situation, the average firing rate (X) has a probability distribution P(X) similar to that of curve 110 (the IHC at rest), but the distribution has been shifted to the right, evidencing a faster mean firing rate.

FIG. 3 further shows, as curve 114, the average firing rate of an auditory nerve fiber as a function of the IHC voltage when the IHC is hyperpolarized, i.e., when the stereocilia 102 have been displaced in the other direction, and the IHC voltage is about -75 mV. As seen in FIG. 3, in such situation, the average firing rate (X) has a probability distribution P(X) much like that of curve 110 (the IHC at rest), but the distribution has been shifted to the left, evidencing a slower mean firing rate.

Next, with reference to FIG. 4, a graph is shown that illustrates the system dynamic range achieved when the stochastic firing of the auditory nerve fibers 108 remains intact. For low energy stimulation, as represented in graph 120, it is seen that the "spike count" (a histogram or "counting" of the number of firings that occur) follows a somewhat S-shaped curve 120' that starts at 0 and saturates, i.e., reaches a maximum firing rate FR_{M}, at energy level E1. For a higher energy acoustic stimulation, as represented in graph 122, the spike count similarly follows a somewhat S-shaped curve 122' that starts near 0 and saturates at energy level E2. For still higher energy acoustic stimulation, as represented in graph 124, the spike count similarly follows an S-shaped curve 124' that starts near 0 and saturates at energy level E3. For even higher energy acoustic stimulation, as represented in graph 126, the spike count similarly follows a somewhat S-shaped curve 126' that starts near 0 and saturates at energy level E4. The system dynamic range is essentially the difference between the S-shaped curves 120' and 126', and is typically on the order of about 120 dB.

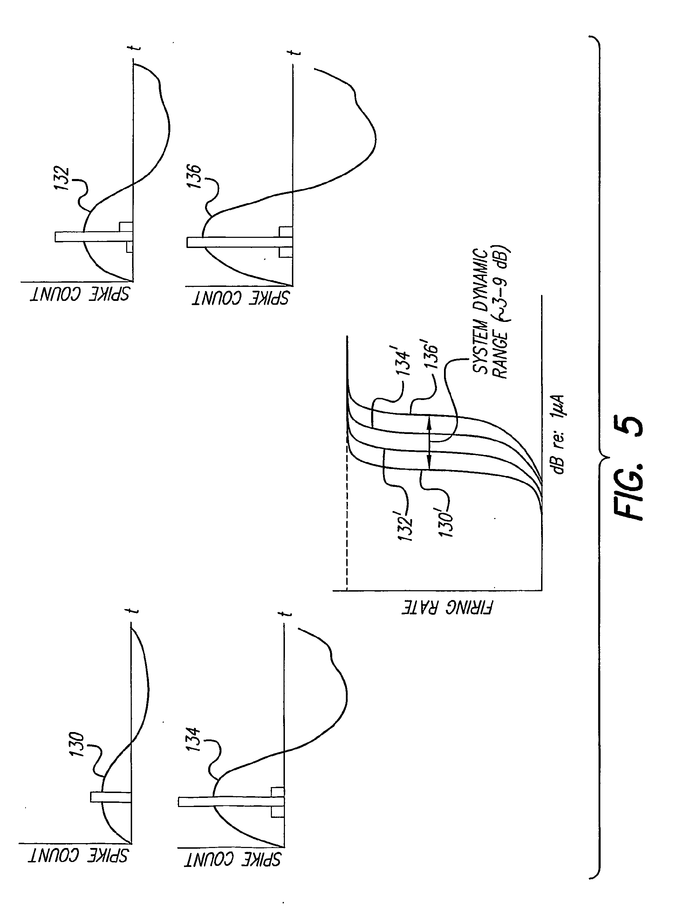

Disadvantageously, the stimulation patterns employed by most neutral stimulators known in the art result in a very narrow system dynamic range for the patient. This is because, as seen in FIG. 5, the electrical stimulation applied to the nerve or muscle is always set to have an amplitude that is at least as great as a measured minium threshold T required for to fire the nerve, so that it will always cause the nerve to fire. Moreover, it is always delivered at a precise time, usually being frequency locked with some type of clock signal that is phase locked, in one form or another, with the frequency of the stimulus signal that is sensed. Thus, as seen in FIG. 5, a low level stimulus, shown in graph 130, which by definition should still be above the minimum threshold T, causes the nerve to fire at a controlled time, e.g., as determined by the system clock signal. The result is a firing-rate curve 130', typical of threshold-based systems, where firing begins to occur only when the threshold is exceeded, and at the rate of the applied stimulus (which, as indicated, is typically frequency-locked to a carrier signal) and the firing rate quickly saturates thereafter at the maximum firing rate, FR_{M}. (The FR_{M} is typically the maximum rate that a given nerve cell is able to follow.) A similar situation occurs as the energy of the applied stimulus increases, all of which energies are above the threshold T, as shown in graphs 132, 134 and 136, resulting in firing-rate curves 132', 134' and 136'. The resulting system dynamic range is very narrow, e.g., on the order of 3-9 dB.

In contrast to the analog approach proposed by Rubinstein et al. (see FIGS. 6A and 6B), the present invention preferably utilizes what will be referred to as an auto-conditioning with high resolution (ACHR) neurostimulation approach. Such ACHR approach does not use an analog signal at all, thereby preserving significant power. The ACHR approach involves generating a high rate pulsatile signal, e.g., a biphasic pulse train having a rate greater than about 2000 Hz (i.e., having a period T less than about 500µS), and having a selected pulse width (PW) within the range of from about 2-3µS (microseconds) to about 100µS. By way of example, the pulse width may be from between about 11 µS to about 21 µS. Generally, it is preferred to make the pulse width as narrow as the particular neural stimulator circuitry will support. The frequency of stimulation, on the other hand, while it should be high, e.g., greater than about 2000 Hz, need not necessarily be much faster than whatever rate is determined as the desired high rate. (As the pulse width narrows, and the frequency or rate remains substantially the same, the duty cycle of the ACHR signal decreases, which helps reduce power consumption.) See FIG. 1 for a definition of T and PW. The ACHR signal is created by amplitude modulating the high rate pulsatile signal with a suitable control signal.

Although not within the scope of the claimed invention, in the case of a cochlear stimulator, the control signal may be the sound information, processed in an appropriate manner, sensed through an external microphone. The frequency of stimulation should be high, e.g. at least 2000 Hz, and preferably 3000 to 5000 Hz, and the pulse widths should be less than about 100 µS.

Although not within the scope of the claimed invention, in the case of a visual prosthetic, the control signal may be visual information, processed in an appropriate manner, sensed through an array of light sensors. Electrical contacts placed in contact or near the retina of the eye apply the ACHR signal to neural fibres within or near the retina. When the neural fibres are stimulated, i.e. when the appropriate nerve fibre synapses fire, information from the nerve fibres that are fired is transmitted to the brain via the optic nerve.

In the case of functional limb movement, the control signal may be a signal that defines the desired movement of the limb, and the electrical contacts through which the ACHR signal is applied are in contact with appropriate muscle tissue or nerve fibres of the limb.

Although not within the scope of the claimed invention, in the case of any other functional recruitment of excitable tissue, the control signal may be a signal that defines the desired biological change that is to occur.

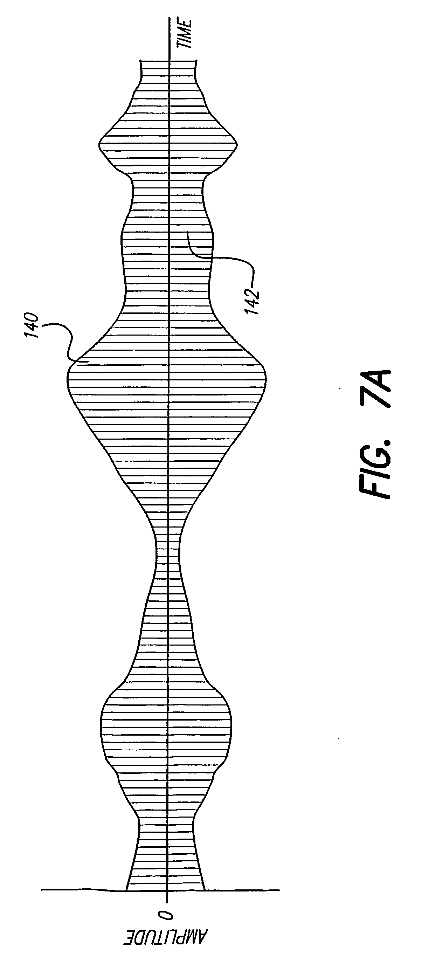

When viewed on a large time scale, e.g. of several milliseconds (mS), the ACHR pulsatile signal provided by a neural stimulator in accordance with the teachings of the present invention would appear as shown in FIG.7A. In FIG. 7A, the horizontal axis is time and the vertical axis is amplitude or magnitude. The relatively slow-varying envelope 140 represents the control information, or control signal, sensed through whatever sensors or other mechanisms are employed to control the neural stimulator, whereas the

vertical lines 142 represent the individual biphasic pulses that are present In the ACHR signal. The horizontal spacing of the vertical lines 142 is not drawn to scale.

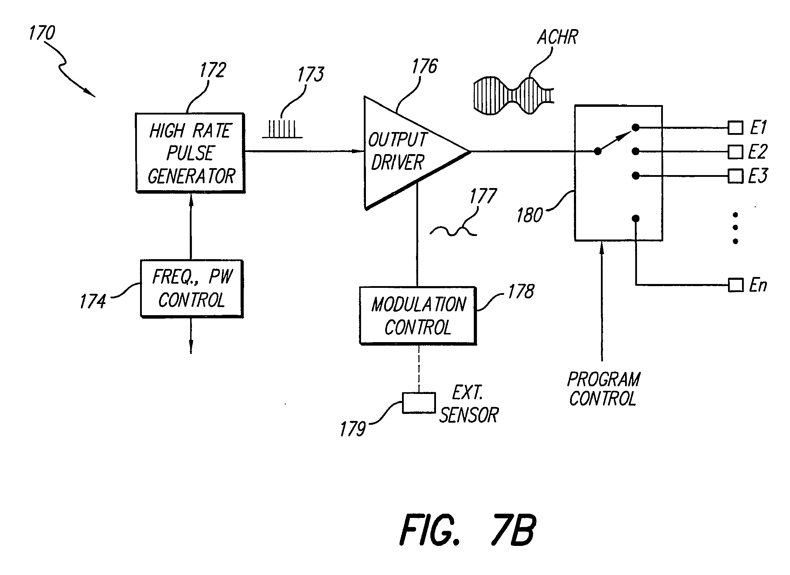

FIG. 7B depicts a functional block diagram of a neurostimulator 170 in accordance with the present invention. The neurostimulator 170 includes a high rate pulse generator 172 that generates a stream of high rate pulses 173 having a rate and pulse width (PW) as controlled by appropriate parameter settings defined by block 174.. A preferred pulse generator is a current pulse generator of the type disclosed in U.S. Patent No. 6,181,969. The high rate pulse stream 173 is directed to an output driver 176. The output driver 176 converts the pulse stream to biphasic pulses, and modulates the amplitude of the biphasic pulses with an appropriate control signal 177. (Alternatively, the pulse generator 172 may be configured to generate a stream of biphasic pulses at the specified rate and pulse width, and the output driver 176 amplitude modulates such pulse stream.) The control signal 177 originates with a modulation control block 178, which in turn may be coupled to an external sensor, e.g., an external microphone, depending upon the particular ACHR application involved.

The ACHR signal generated by the output driver 176 is applied between a selected pair of a multiplicity of electrodes E1, E2, E3, ...En, each of which is in contact with the tissue or nerves to be stimulated, through an output switch 180. The output switch is controlled by appropriate programming signals. When an output current amplifier of the type disclosed in the above-referenced 6,181,969 patent is employed, the output switch 180 is not needed, as each electrode has a programmable current sink/source attached thereto. The ACHR signal may be applied bipolarly between a , selected pair of the multiple electrodes, unipolarly between one of the selected electrodes and a ground electrode, or multipolarly between a first group of the multiple electrodes (functioning a cathode) and a second group of the multiple electrodes (functioning as an anode).

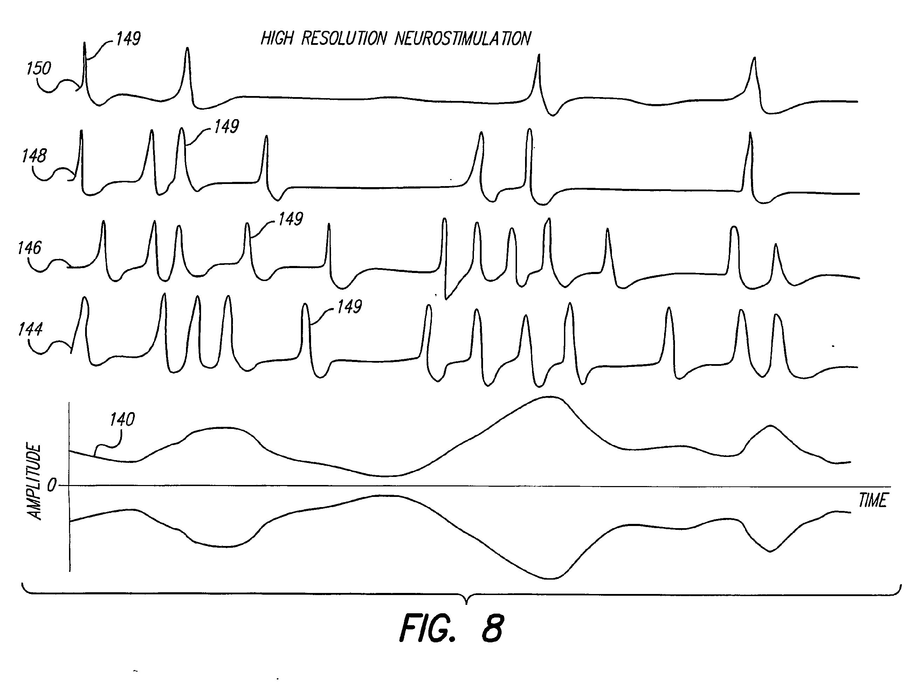

FIG. 8 shows the effect achieved when the ACHR signal of FIG. 7A is applied through multiple electrode contacts to a muscle or nerve cell. The envelope 140 that modulates the amplitude of the ACHR signal is shown at the bottom of FIG. 8. The waveforms 144, 146, 148 and 150 represent neural firings that occur on various ones of the nerve fibers in the population of nerve cells that are stimulated by the ACHR stimulation waveform, with each individual firing being represented by a respective spike 149 in the waveform.

As can be seen in FIG. 8, more than one nerve cell fires as a result of application of the ACHR signal. The firings are stochastic, which better mimics what happens naturally. The more intense the control signal, the more firings that occur. The less intense the control signal, the less firings that occur. Always, however, the firings remain random.

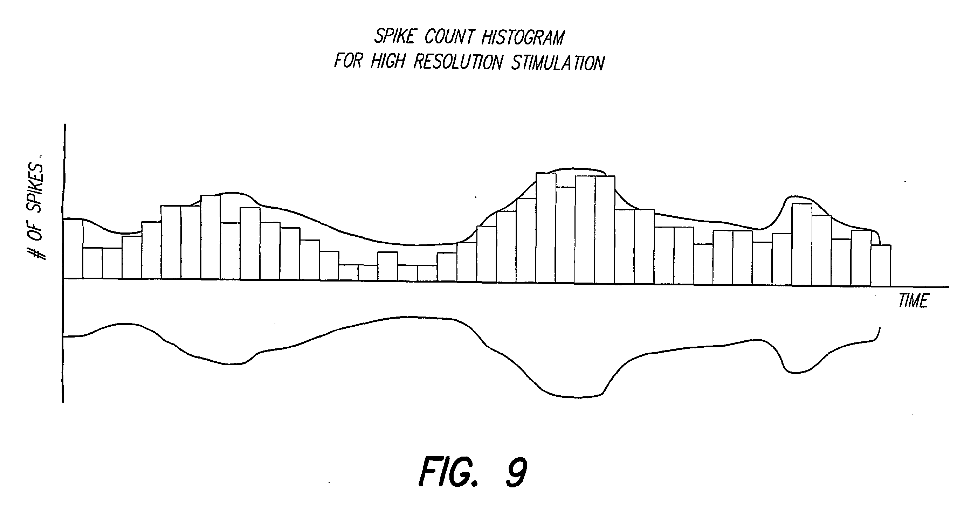

FIG. 9 shows a representative spike count histogram that results from application of the ACHR stimulation waveform to selected nerve fibers. As is evident from FIG. 9, random or stochastic neural firing is achieved, thereby allowing thresholds, dynamic range and psycho physical performance to be enhanced.

Advantageously, a key feature of the invention is that the ACHR signal may be applied to a bundle of muscle or nerve fibers, e.g., to electrically stimulate movement of a limb, and the intensity (or amplitude) of the control signal (the envelope 140 - see FIG. 7A) may be modulated in an appropriate manner so as to bring about the desired movement. That is, the intensity of the control signal may be gradually increased, and then gradually decreased, thereby eliciting gradual recruitment of the muscle excitable tissue or nerves, thereby causing a gradual movement (as opposed to a jerky movement) of the limb.

A neurostimulator suitable for practicing the invention may take many forms, depending upon the particular muscle or nerves that are to be stimulated. So long as the neurostimulator has the capacity to generate a high frequency pulsatile signal, with the ability to modulate the intensity of the individual pulses within the signal, it could be satisfactorily used to practice the invention.

A representative neurostimulator suitable for auditory nerve stimulation Is disclosed in U.S. Patents 6,219,580 or 6,067,474.

A representative neurostimulator suitable for stimulating the nerves of the spinal cord is disclosed in U.S. Patent Application Serial No. 09/626,010, filed 7/26/2000.

The neurostimulator disclosed in the '010 patent application may be easily adapted or modified in order to apply the invention to muscle stimulation, e.g., functional electrical stimulation (FES) for effecting the movement of limbs or for other purposes.

As described above, it is seen that through the proper use of a neurostimulator, i.e., by generating an appropriate high frequency pulsitile signal that is amplitude-modulated with an appropriate control signal, it is possible to have populations of neuron fibers be stimulated at a rate that is faster than an individual neuron fiber is able to follow. Advantageously, such fast stimulation results in a randomization of interspike intervals, or a randomization of when the individual neuron fibers fire. When the neuron is no longer phase-locked to the carrier pulse, its firing probability becomes a function of stimulus energy, and thus becomes more like "natural" neural firing. Such randomization in a neural population better enables the population of neuron fibers to encode the fine details of the desired biological function that is being controlled. That is, the population of neuron fibers is able to encode what a single neuron fiber is not able to encode.

Further, as described above, it is seen that by restoring stochastic firing to the selected nerves, thresholds, dynamic range and psycho physical performance are significantly enhanced.

While the invention herein disclosed has been described by means of specific embodiments and applications thereof, numerous modifications and variations could be made thereto by those skilled in the art without departing from the scope of the invention set forth in the claims.

## Claims

1. A neurostimulator for stimulating muscle or neural excitable tissue, the neurostimulator having multiple electrode contacts through which electrical stimulation may be applied to muscle or neural tissue, the neurostimulator comprising:
means for generating a pulsatile stimulation waveform having a pulse rate sufficiently fast and a pulse width sufficiently narrow to induce stochastic neural firing within muscle or nerve excitable tissue;
means for amplitude modulating the pulsatile stimulation waveform with control information representative of a mean neural firing rate for the muscle or nerve excitable tissue to achieve a desired function; and
means for applying the amplitude-modulated pulsatile stimulation waveform to selected ones of the multiple electrode contacts, whereby excitable tissue is stochastically stimulated, wherein the neurostimulator comprises a functional electrical stimulator having multiple electrical contacts adapted to contact muscle and neural tissue of a limb, and **characterized in that** the functional electrical stimulator includes means for defining desired limb movement, and wherein said desired limb movement is used to amplitude modulate the pulsatile stimulation waveform, which amplitude-modulated pulsatile stimulation waveform is applied through the electrical contacts for the purpose of eliciting stochastic neural firing of excitable muscle and neural tissue located in or near the limb to be moved.

2. The neurostimulator of claim 1, wherein the pulse rate of the pulsatile stimulation waveform varies from 2000 Hz to 5000 Hz.

3. The neurostimulator of claim 2, wherein the pulse widths of the pulsatile stimulation waveform vary from about 2µS to 100µS.

## Patentansprüche

1. Neurostimulator zum Stimulieren von erregbarem Muskel- oder Nervengewebe, wobei der Neurostimulator multiple Elektrodenkontakte aufweist, über die eine elektrische Stimulation auf das Muskel- oder Nervengewebe aufgebracht werden kann, wobei der Neurostimulator Folgendes umfasst:
Mittel zum Erzeugen einer pulsatilen Stimulations-Wellenfonn mit einer Pulsrate, die zur Induktion von stochastischem neuronalem Feuern im erregbaren Muskel- oder Nervengewebe ausreichend schnell ist und einer Pulsbreite, die dazu ausreichend eng ist;
Mittel zur Amplitudenmodulation der pulsatilen Stimulations-Wellenform mit Kontrollinformationen, die für eine mittlere neuronale reuerungsrate für das erregbare Muskel- oder Nervengewebe zum Erreichen einer gewünschten Funktion repräsentativ sind; und
Mittel zum Aufbringen der amplitudemnodulierten pulsatilen Stimulations-Wellenform auf die ausgewählten der multiplen Elektrodenkontakte, wodurch erregbares Gewebe stochastisch stimuliert wird, wobei der Neurostimulator einen funktionellen elektrischen Stimulator mit multiplen elektrischen Kontakten umfasst, die zum Kontaktieren des Muskel- und Nervengewebes einer Extremität angepasst sind und **dadurch gekennzeichnet, dass** der funktionelle elektrische Stimulator Mittel zur Definition der gewünschten Bewegung der Extremität einschließt, und worin die gewünschte Bewegung der Extremität zur Modulation der Amplitude der pulsatilen Stimulations-Wcllenform verwendet wird, welche amplitudenmodulierte pulsatile Stimulations-Wellenform über die elektrischen Kontakte zum Zweck des Auslösens des stochastischen neuronalen Feuerns im erregbaren Muskel- und Nervengewebe aufgebracht wird, das sich in oder in der Nähe der Extremität, die bewegt werden soll, befindet.

2. Neurostimulator nach Anspruch 1, worin die Pulsrate der pulsatilen Stimulations-Wellenform von 2000 Hz bis 5000 Hz variiert.

3. Neurostimulator nach Anspruch 2, worin die Pulsbreiten der pulsatilen Stimulations-Wellenform von ca. 2 µs bis 100 µs variieren.

## Revendications

1. Un neurostimulateur destiné à la stimulation de tissu musculaire ou nerveux excitables, le neurostimulateur ayant des contacts à électrodes multiples par le biais desquels une stimulation électrique peut être appliquée au tissu musculaire ou nerveux, le neurostimulateur comprenant :
un moyen de génération d'une forme d'onde de stimulation pulsatile ayant un taux d'impulsion suffisamment rapide et une largeur d'impulsion suffisamment étroite pour induire une stimulation nerveuse stochastique dans le tissu musculaire ou nerveux excitable ;
un moyen de modulation de l'amplitude de la forme d'onde de stimulation pulsatile avec des informations de commande représentatives d'un taux de stimulation du tissu nerveux moyen pour que le tissu musculaire ou nerveux excitable réalise une fonction désirée ; et
un moyen d'application de la forme d'onde de stimulation pulsatile à modulation d'amplitude à des contacts sélectionnés parmi les contacts à électrodes multiples, par lequel le tissu excitable est stochastiquement stimulé, selon lequel le neurostimulateur comprend un stimulateur électrique fonctionnel ayant des contacts électriques multiples adaptés pour toucher le tissu musculaire et nerveux d'un membre et **caractérisé en ce que** le stimulateur électrique fonctionnel comprend un moyen de définition du mouvement souhaité du membre et selon lequel ledit mouvement souhaité du membre est utilisé pour moduler l'amplitude de la forme d'onde de stimulation pulsatile, laquelle forme d'onde de stimulation pulsatile à modulation d'amplitude est appliquée par le biais des contacts électriques dans le but de provoquer une stimulation nerveuse stochastique dans le tissu musculaire et nerveux excitable dans ou à proximité du membre à déplacer.

2. Neurostimulateur de la revendication 1, selon lequel le taux d'impulsion de la forme d'onde de stimulation pulsatile varie de 20001-lz à 5000 Hz.

3. Neurostimulateur de la revendication 2, selon lequel les largeurs d'impulsions de la forme d'onde de stimulation pulsatile varient d'environ 2 µs à 100 µs.
